(19) **Europäisches Patentamt / European Patent Office / Office européen des brevets**

(11) **EP 3 141 581 B1**

(12) # EUROPEAN PATENT SPECIFICATION

(45) Date of publication and mention
of the grant of the patent:
**05.11.2025 Bulletin 2025/45**

(21) Application number: **15789902.2**

(22) Date of filing: **28.04.2015**

(51) International Patent Classification (IPC):
**A61K 6/891** (2020.01)    **A61K 6/76** (2020.01)

(52) Cooperative Patent Classification (CPC):
**A61K 6/76; C08K 3/36; A61K 6/891; C08J 3/20;
C08K 3/00; C08K 9/06; C08L 71/10**

(86) International application number:
**PCT/JP2015/062858**

(87) International publication number:
**WO 2015/170649 (12.11.2015 Gazette 2015/45)**

(54) **RESIN COMPOSITE MATERIAL AND METHOD FOR MANUFACTURING RESIN COMPOSITE MATERIAL**

HARZVERBUNDMATERIAL UND VERFAHREN ZUR HERSTELLUNG EINES HARZVERBUNDMATERIALS

MATÉRIAU COMPOSITE À BASE DE RÉSINE ET PROCÉDÉ DE FABRICATION D'UN MATÉRIAU COMPOSITE À BASE DE RÉSINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.05.2014 JP 2014096043**

(43) Date of publication of application:
**15.03.2017 Bulletin 2017/11**

(73) Proprietor: **Tokuyama Dental Corporation
Tokyo 110-0016 (JP)**

(72) Inventors:
• **YAMAGAWA, Junichiro
Tokyo 110-0016 (JP)**

• **SHIMIZU, Tomonao
Tokyo 110-0016 (JP)**

(74) Representative: **Gill Jennings & Every LLP
The Broadgate Tower
20 Primrose Street
London EC2A 2ES (GB)**

(56) References cited:
**WO-A1-2011/004892    WO-A1-2013/088921
WO-A1-2013/089078    US-A1- 2012 100 365
US-A1- 2013 252 497    US-A1- 2013 252 497
US-A1- 2014 275 398**

## Description

Technical Field

The present invention relates to a resin composite material and a method of manufacturing a resin composite material.

Background Art

Super engineering plastics are used in a wide range of applications in, for example, an electrical and electronic field, an aerospace field, an automotive industry, a medical field, and a general industrial field. Of the super engineering plastics, a polyaryletherketone resin is considered to be particularly promising by virtue of its excellent chemical properties and physical properties. Although the super engineering plastics have various useful properties, there is also a problem in that the super engineering plastics are liable to be deflected or abraded. A generally known method of ameliorating the problem involves adding a filler to a resin to provide a resin composite material.

Meanwhile, a dental material to be used for the purpose of, for example, restoring a defective portion of a tooth is required to have high mechanical strength. Accordingly, from the viewpoint of securing the mechanical strength or the like, as such dental material, in general, there is widely utilized a resin composite material for dental use obtained by adding any of various fillers to a radicallypolymerizable monomer, such as a (meth) acrylate-based polymerizable monomer (for example, Patent Literature 1). In addition, there is also a proposal of a resin composite material obtained by adding any of various fillers to a super engineering plastic (for example, Patent Literature 2).

US 2013/0252497 A1 (Patent Literature 3) discloses a thermoplastic fiber comprising a core constructed of a first material, a shell positioned to surround the core and constructed of a second material, and magnetic particles that are ferromagnetic and/or ferrimagnetic particles where the particles are either mainly, almost exclusively, or exclusively arranged in the shell. US 2012/0100365 A1 (Patent Literature 4) discloses a thermoplastic resin composition of which the molding efficiency can be improved by a high flowability and a high crystallization temperature while ensuring desired physical properties in a molded product.

US 2014/0275398 A1 (Patent Literature 5) discloses a PEEK resin composition used to injection mold tubes, whilst WO 2013/088921 A1 (Patent Literature 6) provides a high-mechanical-strength organic/inorganic composite wherein a resin with a high melt viscosity contains highly dispersed particles with a high filling fraction.

Citation List

Patent Literature

[PTL 1] JP 2011-144121 A
[PTL 2] JP 2013-144778 A
[PTL 3] US 2013/0252497 A1
[PTL 4] US 2012/0100365 A1
[PTL 5] US 2014/0275398 A1
[PTL 6] WO 2013/088921 A1

Summary of Invention

Technical Problem

Those resin composite materials each have high bending strength and high rigidity. However, a resin composite material to be utilized as a mechanical part, a structural material, or a dental material is often required to have further excellent mechanical characteristics. In order to meet such requirement, for example, to obtain higher rigidity or abrasion resistance, it is effective to blend a filler formed of inorganic particles at a high ratio into a resin. However, such resin composite material is liable to be brittle, and the brittleness causes breakage or a similar phenomenon (chipping or the like) when a certain strain is applied to the resin composite material.

The present invention has been made in view of the above-mentioned circumstances, and an object of the present invention is to provide a resin composite material that has more excellent toughness than that of a related-art resin composite material, and hence, while having rigidity, does not undergo breakage even when a certain strain is applied thereto, and a method of manufacturing the same.

Solution to Problem

The above-mentioned object is achieved by a resin composite material according to claim 1.

In a resin composite material of the present invention, it is preferred that (B) the inorganic particles include silica-based inorganic particles.

In a resin composite material of the present invention, it is preferred that (B) the inorganic particles be surface-treated with a silane coupling agent.

In a resin composite material of the present invention, it is preferred that the resin composite material further include a pigment.

In a resin composite material of the present invention, it is preferred that the resin composite material be produced by: feeding raw materials including (A) the polyaryletherketone resin and (B) the inorganic particles from a raw material feeding port of a melt-kneading apparatus including a barrel having the raw material feeding port and a screw rotatably arranged in the barrel; and melt-kneading the raw materials so that the following expression (I) is satisfied:

$$500 \leq (\pi \times D \times R)/X \qquad \text{Numerical Expression (I)}$$

in the numerical expression (I), D represents an outer diameter (mm) of the screw, R represents a number of rotations (1/s) of the screw, and X represents a width (mm) of a narrowest portion in a gap between an inner peripheral surface of the barrel and the screw.

A method of manufacturing a resin composite material according to the present invention is according to claim 6.

Advantageous Effects of Invention

According to the present invention, the resin composite material that has more excellent rigidity and toughness than those of the related-art resin composite material, and the method of manufacturing the same can be provided.

Brief Description of Drawings

FIG. 1 is a schematic cross-sectional view of an example of the cross-sectional structure of a kneading machine, for describing the numerical expression (I).
FIG. 2 is a schematic cross-sectional view of another example of the cross-sectional structure of a kneading machine, for describing the numerical expression (I).

(Resin Composite Material)

A resin composite material includes: (A) 100 parts by volume of a polyaryletherketone resin having a melt viscosity of from 210 [Pa·s] to 350 [Pa·s] at a temperature of 370°C and a shear rate of 1,220 [1/s]; and (B) 20 parts by volume to 60 parts by volume of inorganic particles. Now, details of the constituent materials for the resin composite material are described.

(A) Polyaryletherketone Resin

In the resin composite material, at least the polyaryletherketone resin is used as a resin material constituting a resin matrix. Herein, as the resin material constituting the resin matrix, the polyaryletherketone resin may be used alone, but as required, another resin may also be used in combination with the polyaryletherketone resin. However, even when the polyaryletherketone resin and another resin are used as a blend, it is necessary, from the viewpoint of securing the mechanical strength of the resin composite material, that the polyaryletherketone resin be used as a main component of the resin material constituting the resin matrix. As used herein, the term "main component" means that the ratio of the polyaryletherketone resin in 100 parts by mass of the resin material constituting the resin matrix is 70 parts by mass or more. The ratio of the polyaryletherketone resin in 100 parts by mass of the resin material constituting the resin matrix is preferably 95 parts by mass or more. In addition, the other resin that may be used in combination with the polyaryletherketone resin is not particularly limited as long as the other resin does not significantly degrade rigidity and toughness. However, from the viewpoint of the ease of securing, for example, the mechanical strength, color tone, and chemical durability of the resin composite material, it is suitable to use, for example, a polyarylate resin, a polycarbonate resin, a polyethylene terephthalate resin, a polyphthalamide resin, a polytetrafluoroethylene resin, or a polyphenylene ether resin.

As described above, in the resin composite material, the polyaryletherketone resin is used as the main resin material constituting the resin matrix. Accordingly, a more excellent mechanical strength than that of a related-art resin composite material can be easily obtained.

The polyaryletherketone resin is a thermoplastic resin containing, in a structural unit thereof, at least an aromatic group, an ether group (ether linkage), and a ketone group (ketone linkage), and often having a linear polymer structure in which benzene rings (phenylene groups) are bonded through the ether group and the ketone group. Typical examples of the polyaryletherketone resin include polyetherketone (PEK), polyetheretherketone (PEEK), polyetherketoneketone (PEKK), and polyetherketoneetherketoneketone (PEKEKK). The aromatic group contained in the structural unit of the polyaryletherketone resin may have a structure having two or more benzene rings, such as a biphenyl structure. In addition, the structural unit of the polyaryletherketone resin may contain a sulfonyl group or any other copolymerizable monomer unit.

From the viewpoints of the color tone and physical properties, it is preferred to use, as the polyaryletherketone resin to be used in the resin composite material, polyetheretherketone having a repeating unit in which the ether group and the ketone group contained in the main chain are arranged in the order "ether-ether-ketone". The polyetheretherketone having a repeating unit is commercially available under, for example, the trade name "PEEK", and specific examples thereof include VESTAKEEP (trademark, Daicel-Evonik Ltd.) and VICTREX PEEK (trademark, Victrex plc.).

(A) The polyaryletherketone resin to be used in the resin composite material has a feature of having a melt viscosity of from 210 [Pa·s] to 350 [Pa·s] at a temperature of 370°C and a shear rate of 1,220 [1/s]. In the present invention, the term "melt viscosity" means a value [Pa·s] for a viscosity measured with a capillary rheometer having a size of 10.0 mm in length and $\varphi$ 1.0 mm in diameter and provided with a mechanism for heating to at least 400°C under a state in which the test speed of a piston is adjusted so as to achieve a test temperature of 370°C and a shear rate of 1,220 [1/s]. Measurement is performed under conditions in accordance with JIS K7199:1999 except for those described above.

The melt viscosity of a resin may be measured under various measurement conditions (temperature and shear rate) as long as a temperature region in which the resin is brought into a molten state is adopted, and the value for the melt viscosity changes depending on the measurement conditions. Meanwhile, the resin composite material is produced through a process of melt-kneading raw materials including (A) the polyaryletherketone resin and (B) the inorganic particles in a molten state. Accordingly, the inventors of the present invention have considered that the mechanical characteristics of the resin composite material are also strongly affected by the molten state of (A) the polyaryletherketone resin during the melt-kneading, and have selected, as measurement conditions for the melt viscosity, a temperature of 370°C and a shear rate of 1,220 [1/s]. That is, the temperature that is a measurement condition for the melt viscosity has been set to 370°C because the temperature is higher than the melting point of a general polyaryletherketone resin, which is about 340°C, by several tens of degrees, and can allow the polyaryletherketone resin to have sufficient fluidity suitable for kneading. In addition, the shear rate that is a measurement condition has a correlation with the shear force to be applied to the raw materials during the melt-kneading. When the shear rate is excessively small, it is difficult to uniformly disperse the inorganic particles in the resin matrix, but even when the shear rate is made higher and higher, it is considered that the dispersibility-improving effect tends to be gradually saturated. Therefore, in order to reliably and stably obtain a resin composite material having excellent mechanical characteristics, a shear rate at a point when the dispersibility-improving effect starts to be gradually saturated while the shear rate is increased is important. Accordingly, from such viewpoint, the shear rate has been set to 1,220 [1/s].

In general, in order to add a high blending amount of a filler, such as inorganic particles, to a thermoplastic resin, a resin having low melt viscosity and high fluidity, that is, a resin having low melt viscosity is selected from the viewpoint of the ease of molding, such as injection molding. However, the present invention has a feature in that the polyaryletherketone resin having the specific melt viscosity is selected and used in order to obtain high toughness. The melt viscosity depends on the temperature and the shear rate. In general, as the temperature becomes higher or the shear rate becomes higher, the melt viscosity becomes smaller.

The reason why high toughness is expressed through the selection of the polyaryletherketone resin having a melt viscosity in the above-mentioned range is unclear. However, the inventors of the present invention presume that the reason is as described below. That is, it is considered that, by virtue of a feature in that the polyaryletherketone resin has a certain viscosity when a shear rate is applied thereto, the dispersion of the inorganic particles in the polyaryletherketone resin serving as the matrix becomes extremely satisfactory, and the microcrystalline structure of the polyaryletherketone resin using the inorganic particles as nuclei is generated in a larger amount, resulting in the expression of high toughness. When the melt viscosity is less than 210 [Pa·s], the dispersion of the inorganic particles as described above does not easily occur, and hence high toughness cannot be obtained. In addition, when the melt viscosity is more than 350 [Pa·s], an increase in viscosity when the inorganic particles are blended into the polyaryletherketone resin becomes remarkable, and hence it is difficult to adjust the resin composite material owing to overload on a kneading apparatus. In addition, shear heat is generated along with an increase in viscosity due to the addition of the inorganic particles during kneading to cause thermal decomposition degradation of the resin component, and hence the toughness is lowered. Further, in molding processing by injection molding, press molding, or the like, the high melt viscosity increases the frequency at which a molding failure occurs, and hence the toughness is lowered. The melt viscosity falls within more preferably the range of from 220 Pa·s to 300 Pa·s, most preferably the range of from 230 Pa·s to 280 Pa·s.

As a suitable commercial product satisfying such melt viscosity, for example, VESTAKEEP ZV7401 (224 Pa·s) or VESTAKEEP 2000G (238 Pa·s) is preferred. In addition, when the resin composite material is used in a dental application, for example, PEEK OPTIMA LT2 (330 Pa·s) or VESTAKEEP M2G (238 Pa·s) is preferred. One kind of the polyaryletherketone resins may be used alone, or two or more kinds thereof may be used in combination.

(B) Inorganic Particles

In the resin composite material, the inorganic particles are blended at a ratio of from 20 parts by volume to 60 parts by volume with respect to 100 parts by volume of the polyaryletherketone resin. The inorganic particles are contained in a state of being dispersed in the polyaryletherketone resin matrix included in the resin composite material. The resin composite material can obtain high rigidity by virtue of including the inorganic particles. When the blending ratio is less than 20 parts by volume, high rigidity cannot be obtained. When the blending ratio is more than 60 parts by volume, brittleness tends to be expressed in the resin composite material to lower its toughness. The blending ratio more preferably falls within the range of from 30 parts by volume to 50 parts by volume. A method of adjusting the volume amount of the inorganic particles may involve determining the true density of the inorganic particles in advance with a specific gravity meter or the like, and measuring the mass of the inorganic particles on a weighing scale so as to achieve a required number of parts by volume, followed by their blending into the polyaryletherketone resin, or may involve determining a relationship between the bulk density or the tap density and the true density of the inorganic particles in advance with a powder physical property measuring machine, a specific gravity meter, or the like, and blending the inorganic particles into the polyaryletherketone resin by supplying the inorganic particles from the inorganic particles filled in a container or the like to a kneading machine under such a condition as to achieve the required number of parts by volume. The shape and internal structure of each of the inorganic particles are not particularly limited, and the inorganic particles may each have any shape and internal structure. However, it is preferred to avoid the use of inorganic particles each having a tube shape or a hollow structure. Preferred examples of the shape of each of the inorganic particles include a spherical shape, an irregular shape, and a whisker shape, and a preferred example of the internal structure is a solid structure.

A material (component) for (B) the inorganic particles is not particularly limited, and specifically, there may be used, for example: silica glass, borosilicate glass, soda glass, aluminosilicate glass, fluoroaluminosilicate glass, and glass containing a heavy metal (such as barium, strontium, or zirconium) ; glass ceramics, such as crystallized glass obtained by depositing a crystal in any such glass, and crystallized glass obtained by depositing a crystal of diopside or leucite; composite inorganic oxides, such as silica-based inorganic particles, e.g., silica-zirconia, silica-titania, and silica-alumina; oxides each obtained by adding a Group I metal oxide to any such composite inorganic oxide; and metal inorganic oxides, such as silica, alumina, titania, and zirconia. When the resin composite material is utilized as a dental material, a material for the inorganic particles is preferably at least one material selected from silica, zirconia, alumina, and titania because of less harmfulness to a living body, a satisfactory color tone, and satisfactory dispersibility. In particular, from the viewpoint of the ease of further improving the rigidity and toughness of the resin composite material, the inorganic particles are preferably silica-based inorganic particles.

In the present invention, the term "silica-based inorganic particles" refers to silica particles, and particles each formed of a composite oxide of silica and, for example, another metal oxide (silica-based composite oxide particles). In the silica-based composite oxide particles, the content of silica is preferably 30 mol% or more, more preferably from 30 mol% to 98 mol%, particularly preferably from 50 mol% to 92 mol% (the content of the other metal oxide is preferably 70 mol% or less, more preferably from 70 mol% to 2 mol%, particularly preferably from 50 mol% to 8 mol%). The content of the other metal oxide in the silica-based composite oxide particles is desirably adopted in consideration of, for example, optical characteristics, such as X-ray transmission/non-transmission properties and fluorescent properties, catalytic characteristics, and ion elution characteristics of the resin composite material in accordance with purposes. Examples of the metal oxide to be composited with silica include $B_2O_3$, $TiO_2$, $SrO$, $BaO$, $ZrO_2$, $HfO_2$, $La_2O_3$, $Y_2O_3$, $ZnO$, $Yb_2O_3$, and $Sb_2O_3$. In addition, in the silica-based composite oxide particles, a metal fluoride or the like may be blended in place of the metal oxide or together with the metal oxide. Similarly, in the present invention, the term "titania-based inorganic particles" refers to titania particles, and particles each formed of a composite oxide of titania and, for example, another metal oxide (titania-based composite oxide particles). Herein, the foregoing description of the silica-based composite oxide particles similarly applies to specific details of the titania-based composite oxide particles except that silica is replaced by titania (provided that the case of using $TiO_2$ as the metal oxide to be composited is excluded).

In the resin composite material, the blending amount (parts by mass) of the inorganic particles with respect to 100 parts by mass of the polyaryletherketone resin may be appropriately selected as long as the blending amount falls within a range falling within the range of from 20 parts by volume to 60 parts by volume of the inorganic particles with respect to 100 parts by volume of the polyaryletherketone resin. In this case, the blending amount (parts by mass) of the inorganic particles with respect to 100 parts by mass of the polyaryletherketone resin varies depending on the specific gravity of the inorganic particles to be blended. When the inorganic particles are silica particles or silica-based inorganic particles having a specific gravity approximately comparable to that of silica, in view of the fact that the specific gravity of a general polyaryletherketone resin is about 1.3 g/cc and the specific gravity of general silica is about 2.2 g/cc, the blending amount of the silica particles (or silica-based inorganic particles) with respect to 100 parts by mass of the polyaryletherketone resin is preferably from 34 parts by mass to 101 parts by mass, more preferably from 51 parts by mass to 85 parts by mass. In addition, when the inorganic particles are titania particles or titania-based inorganic particles having a specific gravity approximately comparable to that of the titania particles, in view of the fact that the specific gravity of a general polyaryletherketone resin is about 1.3 g/cc and the specific gravity of general titania is about 4.0 g/cc, the blending amount of the titania particles (or titania-based inorganic particles) with respect to 100 parts by mass of the polyaryletherketone resin is preferably from 62 parts by mass to 184 parts by mass, more preferably from 92 parts by mass to 154 parts by mass.

The surfaces of (B) the inorganic particles are preferably surface-treated with a surface treatment agent. In this case, the surface treatment is performed in order to improve the dispersibility of the inorganic particles in the polyaryletherketone resin, and the surfaces of the inorganic particles are modified through the surface treatment. A method for such surface treatment is not particularly limited, and a known surface treatment method may be appropriately utilized. A typical example of the surface treatment method is a surface treatment method involving using, as a surface treatment agent (hydrophobizing agent), a silane coupling agent, a zirconate-based coupling agent, an aluminate-based coupling agent, or a titanate-based coupling agent.

A suitable example of the silane coupling agent is represented by the following general formula (II).

$$R^1\text{-}SiR^2mBn \qquad (II)$$

[In the general formula (II), $R^1$ represents an organic group having, at a terminal, an ethylenically unsaturated group, a methyl group, or an aromatic group, and having 2 to 30 atoms constituting a linear moiety, $R^2$ represents a hydrocarbon group having 1 to 6 carbon atoms, and B represents an alkoxy group having a hydrocarbon having 1 to 6 carbon atoms, a halogen group, or an isocyanate group. In the formula, m and n each represent an integer, the sum of m and n is 3, and m represents an integer in the range of from 0 to 2.

In this connection, of the three groups each described as the terminal group of $R^1$, an ethylenically unsaturated group has a risk of being polymerized during kneading to cause an increase in viscosity, resulting in discoloration due to local heat, and hence, from the viewpoint of obtaining higher whiteness, the terminal group is preferably a methyl group or an aromatic group. In particular, the aromatic group is preferred because the aromatic group has a good affinity for (A) the polyaryletherketone resin, and hence improves kneadability to provide a high preventive effect on the discoloration due to the local heat. In addition, the number of aromatic rings constituting the aromatic group is preferably 1. In addition, when the terminal is the aromatic group, the number of atoms constituting the linear moiety of $R^1$ preferably falls within the range of from 8 to 20.

Examples of the ethylenically unsaturated group include: i) an unsaturated aliphatic group including, for example, a vinyl group, a (meth) acrylic group, or a (meth) acrylamide group; and ii) an aromatic group, such as a phenyl group, a phenoxy group, a phenylamino group, a benzophenone group, a hydroxybenzophenone group, a biphenyl group, or a naphthyl group, the aromatic group including a vinyl group, a (meth) acrylic group, or a (meth) acrylamide group as its substituent.

In addition, examples of the aromatic group serving as the terminal group of $R^1$ include: a monocyclic hydrocarbon group having one aromatic ring, such as a phenyl group, a phenoxy group, a phenylamino group, a benzophenone group, or a hydroxybenzophenone group; a non-condensed polycyclic hydrocarbon group having two aromatic rings, such as a biphenyl group; and a condensed polycyclic hydrocarbon group having two aromatic rings, such as a naphthyl group. The non-condensed polycyclic hydrocarbon group means a group in which a carbon atom constituting one aromatic ring is directly bonded to a carbon atom constituting the other aromatic ring. In addition, those aromatic groups do not include a substituent having a polymerizable carbon-carbon double bond, such as a vinyl group, a (meth) acrylic group, or a (meth)acrylamide group.

A typical example of the organic group having 2 to 30 atoms constituting the linear moiety in $R^1$ is a linear alkylene group having 2 to 20 carbon atoms. The carbon atoms constituting the linear moiety in the $R^1$ include the carbon atoms of the ethylenically unsaturated group, the methyl group, or the aromatic group described as the terminal.

Further, examples of the hydrocarbon group having 1 to 6 carbon atoms of $R^2$ include hydrocarbon groups such as a methyl group, an ethyl group, and a propyl group. Of the three groups each described as B, from the viewpoint of the ease of reaction control, an alkoxy group is preferred. In addition, examples of the alkoxy group having a hydrocarbon having 1 to 6 carbon atoms include a methoxy group and an ethoxy group, and examples of the halogen group include a chloro group and a bromo group.

Examples of such silane coupling agent may include 11-methacryloyloxyundecyltrimethoxysilane, 11-methacryloyloxyundecylmethyldimethoxysilane, 10-methacryloyloxydecyltrimethoxysilane, 10-methacryloyloxydecylmethyldimethoxysilane, 10-methacryloyloxydecyltrichlorosilane, 8-methacryloyloxyoctyltrimethoxysilane, 8-methacryloyloxyoctylmethyldimethoxysilane, 8-methacryloyloxyoxyldimethylmethoxysilane, 8-methacryloyloxyoctyltrichlorosilane, 6-methacryloyloxyhexylmethyldimethoxysilane, 4-methacryloyloxybutyltrimethoxysilane, γ-methacryloyloxypropyltrimethoxysilane, γ-methacryloyloxypropylmethyldimethoxysilane, γ-methacryloyloxypropyldimethylmethoxysilane, γ-methacryloyloxypropyltrichlorosilane, γ-methacryloyloxypropyltriisocyanatosilane, γ-methacryloyloxypropyldimethylisocyanatosilane, γ-methacryloyloxypropyltriethoxysilane, 3-(4-methacryloyloxyphenyl)propyltrimethoxysilane, 3-(4-methacryloyloxyphenyl)propyltrichlorosilane, 3-(4-methacryloyloxyphenyl)propyltriisocyanatosilane, styrylpropyltrimethoxysilane, 3-(N-styrylmethyl-2-aminoethylamino)-propyltrimethoxysilane, (methacryloyloxymethyl)phenylbutyltrimethoxysilane, O-(methacryloxyethyl)-N-(triethoxysilylpropyl)carbamate, N-(3-methacryloxy-2-hydroxypropyl)-3-aminopropyltriethoxysilan e, and acrylate compounds corresponding to these methacrylate compounds, phenoxypropyltrichlorosilane, phenoxypropylmethyldichlorosilane, phenoxypropyldimethylchlorosilane, benzoylpropyltrimethoxysilane, phenylaminopropyltrimethoxysilane, 3-phenylpropylmethyldichlorosilane, 4-phenylbutyltrichlorosilane, 4-phenylbutylmethyldichlorosilane, 11-phenoxyundecyltrichlorosilane, 2-hydroxy-4-(3-triethoxysilylpropoxy)diphenyl ketone, 6-phenylhexyldimethylchlorosilane, N-1-phenylethyl-N'-triethoxysilylpropylurea, 3-(4-methacryloyloxyphenyl)propyltrimethoxysilane, 3-(4-methacryloyloxyphenyl)propyltrichlorosilane, 3-(4-methacryloyloxyphenyl)propyltriisocyanatosilane, styrylpropyltrimethoxysilane, 3-(N-styrylmethyl-2-aminoethylamino)-propyltrimethoxysilane, and (methacryloyloxymethyl)phenylbutyltrimethoxysilane.

Through the surface treatment of the inorganic particles with the surface treatment agent, such as the silane coupling agent, as exemplified above, an affinity between the inorganic particles and the resin component, such as the polyaryletherketone resin, constituting the resin matrix is improved. Accordingly, the dispersibility of the inorganic particles in the resin component can be improved to more effectively disperse a stress, and besides, crystallization of the polyaryletherketone resin is promoted to facilitate the enhancement of toughness. In addition, during the kneading of the resin component and the particle component, friction at a particle interface is reduced, and degradation by a thermal decomposition reaction of the resin component due to local heat generation caused by an increase in viscosity during the kneading can be suppressed, and hence a resin composite material having high toughness can be easily obtained.

One kind of the surface treatment agents may be used alone, or two or more kinds thereof may be used as a mixture. In addition, the amount of the surface treatment agent to be used when the inorganic particles are surface-treated is not particularly limited. However, from the viewpoints of handleability and physical properties of the inorganic particles, the amount is suitably set to the range of from 1 part by mass to 10 parts by mass per 100 parts by mass of the inorganic particles to be surface-treated.

The volume-average particle diameter of (B) the inorganic particles falls within the range of from 0.01 μm to 10 μm. When the volume-average particle diameter is less than 0.01 μm, aggregation between the particles is difficult to disintegrate, and besides, an increase in viscosity is liable to occur when the inorganic particles are blended into the resin, and hence it tends to be difficult to increase the blending amount of the inorganic particles. In addition, when the volume-average particle diameter is more than 10 μm, the absolute number of the inorganic particles in the resin matrix is reduced, and hence high toughness tends to be difficult to express. The volume-average particle diameter preferably falls within the range of from 0.07 μm to 5 μm, and the volume-average particle diameter more preferably falls within the range of from 0.1 μm to 1.0 μm.

Herein, the volume-average particle diameter may be measured using a laser scattering method (using LS230 manufactured by Beckman Coulter, Inc. as a measurement apparatus, and using ethanol as a dispersion medium). For the measurement, 0.01 g to 1 g of a measurement sample is added into 5 ml of ethanol serving as a dispersion medium. The liquid having suspended therein the sample is subjected to dispersion treatment with an ultrasonic disperser for from about 1 minute to about 5 minutes, and the particle size distribution of particles each having a particle diameter in the range of from 0.04 μm to 2,000 μm is measured. On the basis of the thus measured particle size distribution, for divided particle size ranges (channels), a cumulative distribution of volume is drawn from a small diameter side, and a particle diameter at a cumulative percentage of 50% is defined as the volume-average particle diameter (D50).

(Other Components)

Other components may be appropriately added to the resin composite material. For example, an antistatic material, a UV absorber, an X-ray contrast material, a pigment, and the like may be added.

(Application of Resin Composite Material)

The resin composite material may be used as a mechanical part or a structural material. When the resin composite material is used as a dental material, the resin composite material is suitably used for, for example, a dental prosthesis, an artificial tooth, a dental plate, a dental implant (fixture, abutment, or superstructure), a dental crown/bridge estoration material, or an abutment construction material. In view of its characteristic of hardly causing breakage or chipping while having high rigidity, the resin composite material is particularly suitably used as a dental implant or a dental crown/bridge restoration material. When the resin composite material is used in a dental application, its suitable shape is the shape of a final product, such as a dental prosthesis, an artificial tooth, a dental plate, a dental implant (fixture, abutment, or superstructure), a dental crown/bridge restoration material (crown or bridge), or an abutment construction material, . The size of such resin composite material for dental use is generally from 5 mm to 120 mm on a side, and is selected appropriately depending on the application of dental restoration.

In addition, when the resin composite material is used as a dental material, for the purpose of improving its aesthetic appearance, it is preferred that a pigment be added to the resin composite material as another component thereof. In an application where the color tone of the gums is reproduced, such as a dental prosthesis or a dental plate, a red pigment is preferably used. In addition, in an application where the color tone of a tooth is reproduced or the color tone of the base of a tooth is formed, such as an artificial tooth, an abutment, a superstructure, a dental crown/bridge restoration material, or an abutment construction material, a red or yellow pigment is preferably used. Further, for fine adjustment of a color tone, a white pigment, a blue pigment, or the like is appropriately selected and used as required.

(Method of Manufacturing Resin Composite Material)

The resin composite material is manufactured through a melt-kneading step of blending (B) the inorganic particles into (A) the polyaryletherketone resin that has been heated and melted, followed by melt-kneading. A heating and melting temperature is selected from temperatures equal to or higher than the melting point of (A) the polyaryletherketone resin, and preferably selected from the range of from 350°C to 450°C. Any known melt-kneading apparatus may be used as an apparatus to be used in the melt-kneading step without any particular limitation, and for example, a mixer with a heating device, a single-screw melt-kneading apparatus, a twin-screw melt-kneading apparatus, or a kneader may be used. As the apparatus to be used in the melt-kneading step, a melt-kneading apparatus (extrusion molding apparatus) including a barrel having a raw material feeding port and a screw rotatably arranged in the barrel, such as a single-screw melt-kneading apparatus (single-screw extrusion molding apparatus) or a twin-screw melt-kneading apparatus (twin-screw extrusion molding apparatus), is preferably used. Of the various extrusion molding apparatus, in particular, a twin-screw extrusion molding apparatus is more preferably used. When the extrusion molding apparatus is used, the melt-kneading step is performed by feeding raw materials including the polyaryletherketone resin and the inorganic particles from the raw material feeding port and melt-kneading the raw materials. The specifications of the extrusion molding apparatus to be used, its screw shape, and operational conditions may be arbitrarily selected in accordance with purposes. However, the melt-kneading step is preferably performed so that a condition represented by the following numerical expression (I) is satisfied.

$$500 \leq (\pi \times D \times R)/X \qquad \text{Numerical Expression (I)}$$

In the numerical expression (I), $\pi$ represents the ratio of a circle's circumference to its diameter, D represents the outer diameter (mm) of a screw, R represents the number of rotations (1/s) of the screw, and X represents the width (mm) of the narrowest portion in a gap between the inner peripheral surface of a barrel and the screw. More precisely, the outer diameter D (mm) means a value twice as large as the maximum radius in a cross-section orthogonal to the axial direction of the screw. In addition, when the outer diameter of the screw changes in the axial direction of the screw, the outer diameter D in the expression (I) means the outer diameter of the screw at the width X of the narrowest portion.

The extrusion molding apparatus generally includes: a raw material feeding port called a hopper on the outer peripheral surface of a cylindrical barrel on one end side thereof; a die arranged on the other end side of the barrel; and a heating member, such as a heater, arranged on the outer periphery side of the barrel. In addition, one end side of a screw arranged in the barrel (end side of the barrel on the side on which the hopper is arranged) is connected to a driving device, such as a motor, through intermediation of a gear or the like. In the melt-kneading step using a twin-screw extrusion molding apparatus, the raw materials fed from the hopper are, while being melted in the barrel and kneaded between the inner peripheral surface of the barrel and the surface of the rotating screw, moved in the barrel from the hopper side toward the die side. At this time, at the narrowest portion at which the gap between the inner peripheral surface of the barrel and the screw is smallest, the largest shear force is applied to amelt-kneaded product.

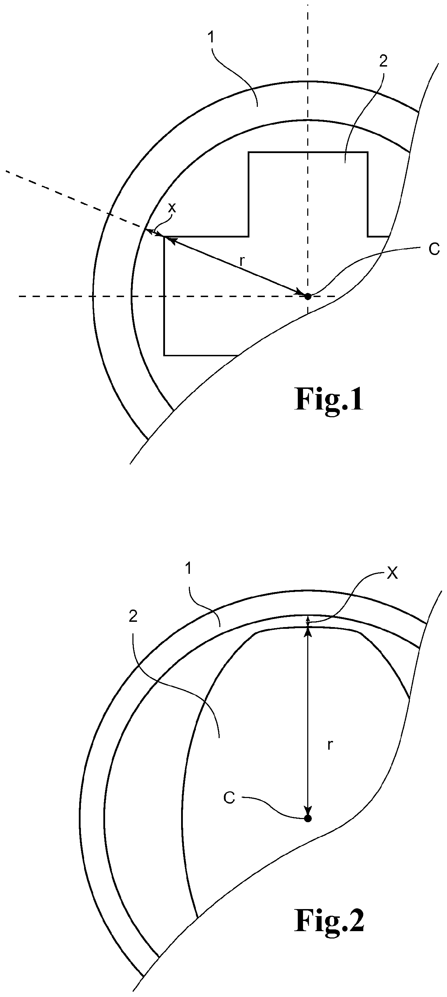

FIG. 1 and FIG. 2 are each a schematic cross-sectional view of an example of the cross-sectional structure of a kneading machine, for describing the numerical expression (I), and specifically, are each an illustration of an example of the cross-sectional structure of an extrusion molding apparatus cut by a plane orthogonal to the axial direction of the screw at the narrowest portion at which the gap between the inner peripheral surface of the barrel and the screw is smallest. In each of the examples illustrated in FIG. 1 and FIG. 2, a screw 2 is arranged in a cylindrical barrel 1. In this case, the outer diameter D of the screw 2 corresponds to a value twice as large as a maximum radius r, which is the distance between a central axis C of the screw 2 and the outermost edge of the screw 2. In addition, the width X of the narrowest portion corresponds to the shortest distance between the outermost edge of the screw 2 and the inner peripheral surface of the barrel 1. The cross-sectional shape of the screw 2 is not limited to a cross shape as exemplified in FIG. 1 or an approximately elliptical shape as exemplified in FIG. 2, and the cross-sectional shape of a known screw may be appropriately selected.

A value S ($=\pi \times D \times R/X$) shown on the right side of the numerical expression (I) corresponds to a shear force to be applied to the raw materials that are being melt-kneaded in the barrel, and a larger value S means that a higher shear force is applied to the raw materials. Herein, when the extrusion molding apparatus is of a single-screw type, the extrusion molding apparatus includes one screw 2, and hence has the value S only for the one screw 2. Meanwhile, when the extrusion molding apparatus is of a twin-screw type, the extrusion molding apparatus includes two screws 2, and hence has the value S for each of the screws 2. Therefore, in this case, it is preferred that the value S satisfy the expression (I) for each of the screws 2. When the value S is 500 or more, a sufficient shear force can be easily applied to the raw materials including (A) the polyaryletherketone resin and (B) the inorganic particles, and hence a resin composite material having higher toughness can be easily obtained. The value S falls within more preferably the range of from 1,000 to 10,000, most preferably the range of from 1,500 to 7,000. When the value S is more than 10,000, it may become difficult to adjust the resin composite material owing to overload on the kneading apparatus. In addition, when the value S is excessively large, shear heat is increased and thermal decomposition degradation of the resin component is liable to occur, with the result that toughness is liable to be lowered in some cases. In the range of the value S of from 1,500 to 7,000, the lower limit value is more preferably 1,530 or more, still more preferably 2,000 or more, most preferably 2,500 or more, and the upper limit value is more preferably 6,000 or less, still more preferably 5,500 or less.

When the resin composite material is manufactured, it is preferred that, as described above, the melt-kneading step be performed using the extrusion molding apparatus so that the numerical expression (I) is satisfied. However, if a shear force substantially equivalent to that in the case represented by the numerical expression (I) can be applied to the raw materials in the melt-kneading step, even when the melt-kneading step is performed using a melt-kneading apparatus other than the extrusion molding apparatus, it is extremely easy to obtain a resin composite material having mechanical characteristics comparable to those in the case where the melt-kneading step is performed with the extrusion molding apparatus so that numerical expression (I) is satisfied.

After the melt-kneading step, various post-processes maybe carried out as required. For example, themelt-kneadedproduct in a high-temperature state immediately after the melt-kneading step may be directly subjected to injection molding, extrusion molding, or the like to be molded into a predetermined shape. In addition, the melt-kneaded product in a high-temperature state immediately after the melt-kneading step may be first molded into a member for secondary processing having a pellet shape, a powder shape, a block shape, or the like, and then the member for secondary processing may be further subjected to any of various types of processing, such as injection molding, extrusion molding, laser forming, cutting processing, machining processing, and polishing processing.

In the manufacture of the resin compositematerial, after the melt-kneading step, in general, a molded body having a predetermined shape is obtained by any of various molding methods, such as injection molding, extrusion molding, and compression molding. At this time, the productivity of the molded body can be enhanced by performing rapid cooling in the mold. In such case, the rapid cooling may cause a residual stress inside the molded body. In addition, the crystal structure of the polyaryletherketone resin serving as the thermoplastic crystalline resin may not be formed in an ideal manner. In order to solve those problems, in the method of manufacturing the resin composite material, the obtained molded body may be subjected to heat treatment as required. When the heat treatment is performed, a residual stress inside the molded body can be released. In addition, the recrystallization of the polyaryletherketone resin vitrified by the rapid cooling can be promoted by the heat treatment, and as a result, the mechanical strength of the molded body can be enhanced.

A method for the heat treatment (heat treatment step) is not particularly limited, but the temperature is preferably selected from a temperature region of the glass transition temperature or more where the melt viscosity is not exceeded, and is preferably selected from the range of from 150°C to 300°C. The heat treatment time is preferably selected from the range of from 30 minutes to 6 hours. A cooling step after the heat treatment preferably involves leaving the molded body in a heating apparatus, such as an oven, in which the heat treatment has been performed under a state in which its heat source is turned off to return the temperature to room temperature over a period of time of 1 hour or more. In addition, for the same reason, in the manufacture of the resin composite material, when the molded body having a predetermined shape is obtained by a molding method, such as injection molding, extrusion molding, or compression molding, by carrying out the above-mentioned cooling step after the resin composite material has been molded into the predetermined shape, it is also possible to obtain a finished molded article having high strength without performing the heat treatment step.

The bending strength of the resin composite material is preferably 190 MPa or more, more preferably 200 MPa or more, most preferably 220 MPa or more. The upper limit of the bending strength is not particularly limited, but from the viewpoint of practical use, is preferably 500 MPa or less. In addition, the rigidity of the resin composite material is preferably 5.5 GPa or more, more preferably 6.0 GPa or more, most preferably 6.5 GPa or more in terms of bending modulus of elasticity. The upper limit of the bending modulus of elasticity is not particularly limited, but from the viewpoint of practical use, is preferably 30 GPa or less.

The breakage resistance of the resin composite material evaluated by the following method is preferably 40% or more, more preferably 60% or more, still more preferably 80% or more, most preferably 100%. The breakage resistance was evaluated by producing a test piece measuring 2 mm long×2 mm wide×25 mm in length and subjecting the test piece to a three-point bending test with a universal testing machine at a support distance of 20 mm and a crosshead speed of 1 mm/min. The test was performed for each of a total of ten test pieces, and a ratio at which breakage was not caused through the application of a strain by a stroke of 3 mm was expressed as the breakage resistance [%].

Examples

The present invention is hereinafter described in more detail by way of Examples.

First, polyaryletherketone resins used in Examples and Comparative Examples and their abbreviations, inorganic particles used therein and other inorganic particles, and their abbreviations, surface treatment agents used therein and their abbreviations, and sample production methods and evaluation methods are shown below.

(Resin Component)

-Polyaryletherketone Resin-

P1: VESTAKEEP M2G (polyetheretherketone resin, manufactured by Daicel-Evonik Ltd., melt viscosity: 238 Pa·s)
P2: VESTAKEEP ZV7401 (polyetheretherketone resin, manufactured by Daicel-Evonik Ltd., melt viscosity: 224 Pa·s)
P3: VESTAKEEP ZV7402 (polyetheretherketone resin, manufactured by Daicel-Evonik Ltd., melt viscosity: 254 Pa·s)
P4: PEEK OPTIMA LT2 (polyetheretherketone resin, manufactured by Victrex plc., melt viscosity: 330 Pa·s)
P5: VICTREX PEEK 90G (polyetheretherketone resin, manufactured by Victrex plc., melt viscosity: 131 Pa·s)
P6: VESTAKEEP 1000G (polyetheretherketone resin, manufactured by Daicel-Evonik Ltd., melt viscosity: 174 Pa·s)
P7: VESTAKEEP 3300G (polyetheretherketone resin, manufactured by Daicel-Evonik Ltd., melt viscosity: 450 Pa·s)
P8: VICTREX PEEK 381G (polyetheretherketone resin, manufactured by Victrex plc., melt viscosity: 360 Pa·s)
P9: VICTREX PEEK 450G (polyetheretherketone resin, manufactured by Victrex plc., melt viscosity: 452 Pa·s)
P10: VESTAKEEP 4000P (polyetheretherketone resin, manufactured by Daicel-Evonik Ltd., melt viscosity: 539 Pa·s)

(Filler Component)

-Inorganic Particles-

F1: silica (spherical, volume-average particle diameter: 1.0 $\mu$m)
F2: silica (spherical, volume-average particle diameter: 3.8 $\mu$m)
F3: silica (spherical, volume-average particle diameter: 0.07 $\mu$m)
F4: titania (amorphous, volume-average particle diameter: 0.3 $\mu$m)

(Surface Treatment Agent)

MPS: $\gamma$-methacryloxypropyltrimethoxysilane
BPS: benzoylpropyltrimethoxysilane

(Melt Viscosity)

A melt viscosity [Pa·s] was measured using Capilograph 1D (manufactured by Toyo Seiki Seisaku-sho, Ltd.), which was a capillary rheometer having a size of 10.0 mm in length and $\varphi$ 1.0 mm in diameter and provided with a mechanism for heating to at least 400°C under a state in which the test speed of a piston was adjusted so as to achieve a test temperature of 370°C and a shear rate of 1,220 [1/s].

(Measurement of Bending Strength)

A test piece having 2 mm long×2 mm wide×25 mm in length was produced, and subjected to a three-point bending test with Autograph (manufactured by Shimadzu Corporation) at a support distance of 20 mm and a crosshead speed of 1 mm/min. Then, bending strength [MPa] was determined on the basis of JIS T6517.

(Measurement of Breakage Resistance)

A test was performed for each of a total of ten test pieces (2 mm long×2 mm wide×25 mm in length), and a ratio at which breakage was not caused through the application of a strain by a stroke of 3 mm was expressed as breakage resistance [%].

(Measurement of Bending Modulus of Elasticity)

A bending modulus of elasticity was calculated from the gradient of a bending load-deflection curve at a stress of from 5 N to 10 N for a test piece having 2 mm long×2 mm wide×25 mm in length on the basis of a tangent method (JIS K7074).

(Surface Treatment Method for Inorganic Particles)

The above-mentioned various filler components were surface-treated by the following procedure. First, 100 g of inorganic particles and 200 ml of toluene were weighed out and mixed and then dispersed with a homogenizer to primary particles to produce a slurry. Next, the slurry was loaded into a three-necked flask having inserted thereinto a reflux condenser, and then 2.4 g of a surface treatment agent was further added. Subsequently, the solution in the three-necked flask was heated to reflux for 2 hours while being stirred. Subsequently, the solid content was separated from the solution subjected to heating reflux treatment through the use of a centrifugal separator. After that, the solid content was washed with toluene twice, and then dried in a vacuum dryer at 90°C for 10 hours. Thus, surface-treated inorganic particles were obtained.

<Example 1>

100 Parts by volume of (A) the polyaryletherketone resin P1 and 39 parts by volume of (B) the inorganic particles F1 were weighed out, and were loaded into a twin-screw extrusion molding apparatus (outer diameter D of each screw: 14.40 mm, width X of narrowest portion: 0.15 mm, temperature during measurement of outer diameter D and width X: 25°C). Melt-kneading was performed under the conditions of a test temperature of 370°C and a number of rotations R of each screw of 16. 67 s$^{-1}$ (1,000 rpm). After that, the melt-kneaded product was recovered, and subjected to pressure press molding into a plate shape (40 mm long×40 mm wide×2 mm thick) using a hot press moldingmachine, followed by slow cooling to provide a resin composite material. The raw material composition and evaluation results of the resultant resin composite material are shown in Table 1.

<Examples 2 to 15>

Resin composite materials were obtained by the same method as that of Example 1 except that the kinds and blending amounts of (A) the polyaryletherketone resin and (B) the inorganic particles used, and the test conditions were changed to conditions shown in Table 1. The raw material compositions and evaluation results of the resultant resin composite materials are shown in Table 1. In Table 1, for each of Examples 11 to 14 having a different value for $(\pi\times D\times R)/X$ from the value of Example 1, the value for $(\pi\times D\times R)/X$ was adjusted by changing the number of rotations R of each screw in the twin-screw extrusion molding apparatus used in Example 1.

<Examples 16 to 18>

Resin composite materials were obtained by the same method as that of Example 1 except that, in the twin-screw extrusion molding apparatus used, the outer diameter D of each screw and the width X of the narrowest portion were changed to 10.64 mm and 0.18 mm, respectively (temperature during measurement of outer diameter D and width X: 25°C), and further, the number of rotations R of each screw was changed to 8.33 s$^{-1}$ (500 rpm) in each of Examples 16 and 18 and changed to 5.00 s$^{-1}$ (300 rpm) in Example 17. The raw material compositions and evaluation results of the resultant resin composite materials are shown in Table 1. In Table 1, values shown in parentheses in the column " (A) Polyaryletherketone resin" of Example 18 are blending ratios (mass%) on a mass basis when the total amount of the resins is defined as 100 mass%.

<Comparative Example 1>

A resin composite material was obtained by the same method as that of Example 1 except that the polyaryletherketone resin P5 was used as a raw material. The raw material composition and evaluation results of the resultant resin composite material are shown in Table 1. The results of the bending test were that all test pieces fractured, indicating low toughness.

<Comparative Example 2>

A resin composite material was obtained by the same method as that of Example 1 except that the polyaryletherketone resin P6 was used as a raw material. The raw material composition and evaluation results of the resultant resin composite material are shown in Table 1. The results of the bending test were that all test pieces fractured, indicating low toughness.

<Comparative Example 3>

Melt-kneading was performed by the same method as that of Example 1 except that the polyaryletherketone resin P7 was used as a raw material. However, the twin-screw extrusion molding apparatus stopped owing to overload during kneading, and hence a resin composite material was not able to be obtained.

<Comparative Example 4>

A resin composite material was obtained by the same method as that of Example 1 except that the blending amount of the inorganic particles was changed to 15 parts by volume. The raw material composition and evaluation results of the resultant resin composite material are shown in Table 1. The results of the bending test were that the modulus of elasticity was low and the rigidity was poor.

<Comparative Example 5>

A resin composite material was obtained by the same method as that of Example 1 except that the blending amount of the inorganic particles was changed to 89 parts by volume. The raw material composition and evaluation results of the resultant resin composite material are shown in Table 1. The results of the bending test were that all test pieces fractured, indicating low toughness.

<Comparative Examples 6 and 7>

Melt-kneading was performed by the same method as that of Example 1 except that the polyaryletherketone resins P8 and P9 were each used as a raw material. However, the twin-screw extrusion molding apparatus stopped owing to overload during kneading, and hence a resin composite material was not able to be obtained.

[Table 1]

| | (A) Polyaryletherketone resin | | (B) Inorganic particles | | | Blending ratio of (B) inorganic particles with respect to 100parts by volume of (A) polyaryletherketone resin | $(\pi \times D \times R)/X$ [1/s] | Measurement result | | |
|---|---|---|---|---|---|---|---|---|---|---|
| | Kind | Melt viscosity | Kind | Surface treatment agent | Volume-average particle diameter | | | Bending strength [MPa] | Bending modulus of elasticity [Gpa] | Breakage resistance |
| Example 1 | P1 | 238 Pa·s | F1 | MPS | 1.0 μm | 39 parts by volume | 5,025 | 225 | 6.5 | 100% |
| Example 2 | P2 | 224 Pa·s | F1 | MPS | 1.0 μm | 39 parts by volume | 5,025 | 205 | 6.4 | 80% |
| Example 3 | P3 | 254 Pa·s | F1 | MPS | 1.0 μm | 39 parts by volume | 5,025 | 221 | 6.5 | 100% |
| Example 4 | P4 | 330 Pa·s | F1 | MPS | 1.0 μm | 39 parts by volume | 5,025 | 201 | 6.3 | 40% |
| Example 5 | P1 | 238 Pa·s | F1 | MPS | 1.0 μm | 25 parts by volume | 5,025 | 194 | 5.6 | 100% |
| Example 6 | P1 | 238 Pa·s | F1 | MPS | 1.0 μm | 59 parts by volume | 5,025 | 220 | 8.5 | 40% |
| Example 7 | P1 | 238 Pa·s | F1 | None | 1.0 μm | 39 parts by volume | 5,025 | 190 | 6.1 | 40% |
| Example 8 | P1 | 238 Pa·s | F1 | BPS | 1.0 μm | 39 parts by volume | 5,025 | 221 | 6.6 | 100% |
| Example 9 | P1 | 238 Pa·s | F2 | MPS | 3.8 μm | 39 parts by volume | 5,025 | 208 | 6.5 | 80% |
| Example 10 | P1 | 238 Pa·s | F3 | MPS | 0.07 μm | 39 parts by volume | 5,025 | 206 | 6.4 | 70% |
| Example 11 | P1 | 238 Pa·s | F1 | MPS | 1.0 μm | 39 parts by volume | 1,507 | 198 | 6.5 | 50% |
| Example 12 | P1 | 238 Pa·s | F1 | MPS | 1.0 μm | 39 parts by volume | 2,511 | 208 | 6.4 | 80% |
| Example 13 | P1 | 238 Pa·s | F1 | MPS | 1.0 μm | 39 parts by volume | 6,279 | 202 | 6.4 | 60% |
| Example 14 | P1 | 238 Pa·s | F1 | MPS | 1.0 μm | 39 parts by volume | 10, 047 | 191 | 6.6 | 40% |
| Example 15 | P1 | 238 Pa·s | F4 | MPS | 0.3 μm | 25 parts by volume | 5, 025 | 197 | 6.9 | 60% |
| Example 16 | P1 | 238 Pa·s | F1 | MPS | 1.0 μm | 39 parts by volume | 1,546 | 208 | 6.3 | 70% |
| Example 17 | P1 | 238 Pa·s | F1 | MPS | 1.0 μm | 39 parts by volume | 928 | 205 | 6.5 | 40% |
| Example 18 | P1(90)/ P10(10) | 258 Pa·s | F1 | MPS | 1.0 μm | 39 parts by volume | 1,546 | 210 | 6.2 | 90% |
| Comparative Example 1 | P5 | 131 Pa·s | F1 | MPS | 1.0 μm | 39 parts by volume | 5,025 | 176 | 6.3 | 0% |
| Comparative Example 2 | P6 | 174 Pa·s | F1 | MPS | 1.0 μm | 39 parts by volume | 5,025 | 175 | 6.3 | 0% |

EP 3 141 581 B1

13

| | (A) Polyaryletherketone resin | | (B) Inorganic particles | | | Blending ratio of (B) inorganic particles with respect to 100parts by volume of (A) polyaryletherketone resin | $(\pi \times D \times R)/-X$ [1/s] | Measurement result | | |
|---|---|---|---|---|---|---|---|---|---|---|
| | Kind | Melt viscosity | Kind | Surface treatment agent | Volume-average particle diameter | | | Bending strength [MPa] | Bending modulus of elasticity [Gpa] | Breakage resistance |
| Comparative Example 3 | P7 | 450 Pa·s | F1 | MPS | 1.0 μm | 39 parts by volume | 5,025 | - | - | - |
| Comparative Example 4 | P1 | 238 Pa·s | F1 | MPS | 1.0 μm | 15 parts by volume | 5,025 | 175 | 4.1 | 100% |
| Comparative Example 5 | P1 | 238 Pa·s | F1 | MPS | 1.0 μm | 89 parts by volume | 5,025 | 209 | 8.9 | 0% |
| Comparative Example 6 | P8 | 360 Pa·s | F1 | MPS | 1.0 μm | 39 parts by volume | 5,025 | - | - | - |
| Comparative Example 7 | P9 | 452 Pa·s | F1 | MPS | 1.0 μm | 39 parts by volume | 5,025 | - | - | - |

Reference Signs List

1    barrel
2    screw
r    maximum radius of screw 2
X    width of narrowest portion

## Claims

1. A resin composite material for dental use, comprising:

    (A) 100 parts by volume of a polyaryletherketone resin having a melt viscosity of from 210 [Pa·s] to 350 [Pa·s] at a temperature of 370°C and a shear rate of 1,220 [1/s]; and
    (B) 20 parts by volume to 60 parts by volume of inorganic particles;
    wherein (B) the inorganic particles have a volume-average particle diameter of from 0.01 $\mu$m to 10 $\mu$m;
    wherein the melt viscosity and volume-average particle diameter are measured in accordance with the methods described in the description; and
    wherein the resin composite material is in the shape of a dental prosthesis, an artificial tooth, a dental plate, a dental implant, a dental crown or bridge restoration material, or an abutment construction material.

2. A resin composite material for dental use according to claim 1, wherein (B) the inorganic particles comprise silica-based inorganic particles.

3. A resin composite material for dental use according to claim 1 or 2, wherein (B) the inorganic particles are surface-treated with a silane coupling agent.

4. A resin composite material for dental use according to any one of claims 1 to 3, further comprising a pigment.

5. A resin composite material for dental use according to any one of claims 1 to 4, wherein the resin composite material for dental use is produced by:

    feeding raw materials including (A) the polyaryletherketone resin and (B) the inorganic particles from a raw material feeding port of a melt-kneading apparatus including a barrel having the raw material feeding port and a screw rotatably arranged in the barrel; and melt-kneading the raw materials so that the following expression (I) is satisfied:

    $$500 \leq (\pi \times D \times R)/X \qquad \text{Numerical Expression (I)}$$

    in the numerical expression (I), D represents an outer diameter (mm) of the screw, R represents a number of rotations (1/s) of the screw, and X represents a width (mm) of a narrowest portion in a gap between an inner peripheral surface of the barrel and the screw.

6. A method of manufacturing a resin composite material, comprising at least a melt-kneading step including:

    feeding raw materials including (A) 100 parts by volume of a polyaryletherketone resin having a melt viscosity of from 210 [Pa·s] to 350 [Pa·s] at a temperature of 370°C and a shear rate of 1,220 [1/s] and (B) 20 parts by volume to 60 parts by volume of inorganic particles from a raw material feeding port of a melt-kneading apparatus including a barrel having the raw material feeding port and a screw rotatably arranged in the barrel; and melt-kneading the raw materials so that the following expression (I) is satisfied:

    $$500 \leq (\pi \times D \times R)/X \qquad \text{Numerical Expression (I)}$$

    in the numerical expression (I), D represents an outer diameter (mm) of the screw, R represents a number of rotations (1/s) of the screw, and X represents a width (mm) of a narrowest portion in a gap between an inner peripheral surface of the barrel and the screw.

**Patentansprüche**

1. Harzverbundmaterial zur dentalen Verwendung, umfassend:

   (A) 100 Volumenteile eines Polyaryletherketonharzes mit einer Schmelzviskosität von 210 [Pa·s] bis 350 [Pa·s] bei einer Temperatur von 370 °C und einer Scherrate von 1.220 [1/s]; und
   (B) 20 Volumenteile bis 60 Volumenteile anorganische Partikel;
   wobei (B) die anorganischen Partikel einen volumendurchschnittlichen Partikeldurchmesser von 0,01 μm bis 10 μm aufweisen;
   wobei die Schmelzviskosität und der volumendurchschnittliche Partikeldurchmesser gemäß den in der Beschreibung beschriebenen Verfahren gemessen werden; und
   wobei das Harzverbundmaterial die Form einer Zahnprothese, eines künstlichen Zahns, einer Zahnplatte, eines Zahnimplantats, eines Zahnkronen- oder Brückenversorgungsmaterials oder eines Abutment-Konstruktionsmaterials aufweist.

2. Harzverbundmaterial zur dentalen Verwendung nach Anspruch 1, wobei (B) die anorganischen Partikel anorganische Partikel auf Kieselsäurebasis umfassen.

3. Harzverbundmaterial zur dentalen Verwendung nach Anspruch 1 oder 2, wobei (B) die anorganischen Partikel mit einem Silan-Kopplungsmittel oberflächenbehandelt werden.

4. Harzverbundmaterial zur dentalen Verwendung nach einem der Ansprüche 1 bis 3, ferner umfassend ein Pigment.

5. Harzverbundmaterial zur dentalen Verwendung nach einem der Ansprüche 1 bis 4, wobei das Harzverbundmaterial zur dentalen Verwendung hergestellt wird durch:

   Zuführen von Rohmaterial, Einschließen (A) des Polyaryletherketonharzes und (B) der anorganischen Partikel aus einem Rohstoffzufuhranschluss einer Schmelzknetvorrichtung, Einschließen eines Zylinders, das den Rohstoffzufuhranschluss und eine drehbar in dem Zylinder angeordnete Schnecke aufweist; und Schmelzkneten der Rohstoffe, sodass folgender Ausdruck (I) erfüllt ist:

   $$500 \leq (\Pi \times T \times R)/X \qquad \text{Numerischer Ausdruck (I)}$$

   in dem numerischen Ausdruck (I) steht D für einen Außendurchmesser (mm) der Schraube, R steht für eine Anzahl von Umdrehungen (1/s) der Schraube und X steht für eine Breite (mm) eines schmalsten Abschnitts in einem Spalt zwischen einer inneren Peripheriefläche des Zylinders und der Schraube.

6. Verfahren zur Herstellung eines Harzverbundmaterials, umfassend mindestens einen Schmelzknetschritt, einschließlich:

   Zuführen von Rohmaterial, Einschließen (A) von 100 Volumenteilen eines Polyaryletherketonharzes mit einer Schmelzviskosität von 210 [Pa·s] bis 350 [Pa·s] bei einer Temperatur von 370 °C und einer Scherrate von 1.220 [1/s] und (B) von 20 Volumenteilen bis 60 Volumenteilen anorganischer Partikel aus einem Rohstoffzufuhranschluss einer Schmelzknetvorrichtung Einschließen eines Zylinders mit dem Rohstoffzufuhranschluss und einer drehbar in dem Zylinder angeordneten Schnecke; und
   Schmelzkneten der Rohstoffe, sodass folgender Ausdruck (I) erfüllt ist:

   $$500 \leq (\Pi \times T \times R)/X \qquad \text{Numerischer Ausdruck (I)}$$

   in dem numerischen Ausdruck (I) steht D für einen Außendurchmesser (mm) der Schraube, R steht für eine Anzahl von Umdrehungen (1/s) der Schraube und X steht für eine Breite (mm) eines schmalsten Abschnitts in einem Spalt zwischen einer inneren Peripheriefläche des Zylinders und der Schraube.

**Revendications**

1. Matériau composite en résine pour usage dentaire, comprenant :

(A) 100 parties en volume d'une résine de polyaryléthercétone présentant une viscosité à l'état fondu de 210 [Pa·s] à 350 [Pa·s] à une température de 370 °C et un taux de cisaillement de 1220 [1/s] ; et

(B) 20 parties en volume à 60 parties en volume de particules inorganiques ;

dans lequel (B) les particules inorganiques présentent un diamètre moyen de particule en volume de 0,01 μm à 10 μm ;

dans lequel la viscosité à l'état fondu et le diamètre moyen de particule en volume sont mesurés conformément aux procédés décrits dans la description ; et

dans lequel le matériau composite en résine est sous la forme d'une prothèse dentaire, d'une dent artificielle, d'une plaque dentaire, d'un implant dentaire, d'un matériau de restauration de couronne ou de bridge, ou d'un matériau de construction de pilier.

2. Matériau composite en résine pour usage dentaire selon la revendication 1, dans lequel (B) les particules inorganiques comprennent des particules inorganiques à base de silice.

3. Matériau composite en résine pour usage dentaire selon la revendication 1 ou la revendication 2, dans lequel (B) les particules inorganiques sont traitées en surface avec un agent de couplage au silane.

4. Matériau composite en résine pour usage dentaire selon l'une quelconque des revendications 1 à 3, comprenant en outre un pigment.

5. Matériau composite en résine pour usage dentaire selon l'une quelconque des revendications 1 à 4, dans lequel le matériau composite en résine pour usage dentaire est produit par :

l'introduction de matières premières comprenant (A) la résine de polyaryléthercétone et (B) les particules inorganiques par un orifice d'alimentation en matière première d'un appareil de malaxage de matière fondue comprenant un cylindre ayant l'orifice d'alimentation en matière première et une vis disposée de manière rotative dans le cylindre ; et le malaxage à l'état fondu des matières premières de telle sorte que l'expression (I) suivante soit satisfaite :

$$500 \leq (\Pi \times D \times R)/X \qquad \text{Expression numérique (I)}$$

dans l'expression numérique (I), D représente un diamètre externe (mm) de la vis, R représente un nombre de rotations (1/s) de la vis, et X représente une largeur (mm) d'une partie la plus étroite dans un espace entre une surface périphérique interne du cylindre et la vis.

6. Procédé de fabrication d'un matériau composite en résine, comprenant au moins une étape de malaxage à l'état fondu comprenant :

l'introduction de matières premières comprenant (A) 100 parties en volume d'une résine de polyaryléthercétone présentant une viscosité à l'état fondu de 210 [Pa·s] à 350 [Pa·s] à une température de 370 °C et un taux de cisaillement de 1220 [1/s] et (B) 20 parties en volume à 60 parties en volume de particules inorganiques par un orifice d'alimentation en matière première d'un appareil de malaxage de matière fondue comprenant un cylindre ayant l'orifice d'alimentation en matière première et une vis disposée de manière rotative dans le cylindre ; et le malaxage à l'état fondu des matières premières de telle sorte que l'expression (I) suivante soit satisfaite :

$$500 \leq (\Pi \times D \times R)/X \qquad \text{Expression numérique (I)}$$

dans l'expression numérique (I), D représente un diamètre externe (mm) de la vis, R représente un nombre de rotations (1/s) de la vis, et X représente une largeur (mm) d'une partie la plus étroite dans un espace entre une surface périphérique interne du cylindre et la vis.

**Fig.1**

**Fig.2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130252497 A1
- US 20120100365 A1
- US 20140275398 A1
- WO 2013088921 A1
- JP 2011144121 A
- JP 2013144778 A